# EUROPEAN PATENT APPLICATION

(11) **EP 2 781 182 A1**
(43) Date of publication of application: **24.09.2014**
(21) Application number: 11875876.2
(22) Date of filing: 15.11.2011
(51) Int. Cl.: A61B 1/00, A61B 1/04, H04N 5/225

(54) **PHOTOGRAPHIC DEVICE AND PHOTOGRAPHIC SYSTEM**

(71) Applicant: Suzuki, Naoki, Tokyo 110-0005 (JP)
(72) Inventor: SUZUKI, Naoki, Tokyo 110-0005 (JP); HATTORI, Asaki, Tokyo 110-0005 (JP)
(74) Representative: Moore, Graeme Patrick
(86) International application number: PCT/JP2011/076740
(87) International publication number: WO 2013/073061

(57) **Abstract**

Provided is a photographic device including a camera array in which plural photographic units are arrayed on a leading end of an arm formed to be capable of passing through a tubular body which is inserted into a body from outside the body. The camera array is displaceably disposed between an alignment location whereat the plural photographic units are arrayed along an extension of the arm and a deployment location whereat the plural photographic units are arrayed laterally to the arm.

## Description

### TECHNICAL FIELD

The present invention relates to a photographic device and a photographic system capable of photographing the inside of a body.

### BACKGROUND ART

A surgical procedure, namely, what is called laparoscopic surgery, which involves using a visual field in a rigid scope inserted into an abdominal cavity through a small incision made in a body surface, and using a surgical instrument through small incisions made in two other parts in the body surface, thereby to perform surgery, has made its appearance in the 1990s and has come into use for cholecystectomy. Since then, this surgery has gradually found its application also in other surgical areas, which in turn has led to various surgical procedures being currently developed.

However, although the visual field obtained by the rigid scope has improved in image quality as the years have passed (refer to Patent Literature 1 or 2), an image captured by a lens located on the leading end of the rigid scope, in practice, has remained narrow in its own visual field and also remained limited in viewable direction as before and has not undergone much change since the start of rigid scope surgery. The performance of the surgical procedure in a narrow field of view, even now, still causes a frequent occurrence of the malpractice of failing to notice hemorrhage occurring in a region which falls outside the visual field, or of causing damage to soft tissue by the surgical instrument coming in contact with the soft tissue outside the visual field. Under such circumstances, we have attempted to develop a camera system for rigid scope surgery of a new type and robotic surgery, which ensures the widest possible field of view even in the abdominal cavity and allows universal movement of a viewpoint and, moreover, is capable of grasping the intra-abdominal state in real time and in four dimensions (or in time and space) by using virtual reality technology.

### CITATION LIST

### PATENT LITERATURE

Patent Literature 1: Japanese Patent Application Publication No. 2001-045472
Patent Literature 2: Japanese Patent Application Publication No. 2007-135756

### SUMMARY OF INVENTION

### TECHNICAL PROBLEM

The present invention has been made in view of the above circumstances. An object of the present invention is to provide a camera system for rigid scope surgery of a new type and robotic surgery, which ensures the widest possible field of view even in an abdominal cavity and allows universal movement of a viewpoint without entailing physical movement of a camera and, moreover, is capable of grasping the intra-abdominal state in real time and in four dimensions (or in time and space) by applying virtual reality technology.

### SOLUTION TO PROBLEM

Therefore, the present invention provides a photographic device including a camera array in which plural photographic units are arrayed on a leading end of an arm formed to be capable of passing through a tubular body which is inserted into a body from outside the body, wherein the camera array is displaceably disposed between an alignment location whereat the plural photographic units are arrayed along an extension of the arm and a deployment location whereat the plural photographic units are arrayed laterally to the arm.

The leading end of the arm may be provided with the plural camera arrays.

Also, preferably, the plural photographic units are arrayed in an arcuate manner at the deployment location.

Also, the photographic device of the present invention may form a stereoscopic image based on image signals from the two adjacent photographic units.

Further, the photographic device of the present invention may include a three-dimensional model generation means for generating a three-dimensional model of a target object contained in a subject, based on plural subject images at different viewpoints captured by the plural photographic units.

Further, the photographic device of the present invention may be configured as given below; specifically, the camera array has plural movable pieces coupled to each other by SMA actuators, and, when the SMA actuators operate, the adjacent movable pieces are inclined and the movable pieces are bent and curved in an arcuate manner, and the photographic units arranged on the movable pieces are oriented toward a predetermined point toward the center of the arc when the movable pieces are bent and curved.

Also, a photographic system of the present invention includes the above-described photographic device, and an image display device which displays plural images at different viewpoints formed based on image signals from plural photographic units provided in the photographic device.

The photographic system of the present invention may include a stereoscopic image generation means for forming a stereoscopic image based on image signals from the two adjacent photographic units, and a display control means for displaying the stereoscopic image.

Further, the photographic system of the present invention may include a three-dimensional model generation means for generating a three-dimensional model of a target object contained in a subject, based on the plural images at the different viewpoints, and a display control means for displaying an image of the target object as observed from any user-selected viewpoint, based on the three-dimensional model.

### BRIEF DESCRIPTION OF DRAWINGS

Fig. 1 is an explanatory view illustrating in schematic form a camera module in which first to eighth cameras are arrayed.
Fig. 2 is a view of assistance in explaining a photographic device having camera arrays as located at an alignment location.
Fig. 3 is a view of assistance in explaining the photographic device with the camera arrays as deployed outward.
Fig. 4 is a view of assistance in explaining the photographic device with the camera arrays as located at a deployment location.
Fig. 5 is a view of assistance in explaining the photographic device with the camera arrays as located at the deployment location.
Fig. 6 is a perspective view of a movable piece and its surroundings as obliquely observed.
Fig. 7 is a cross-sectional view of the movable piece taken in a direction substantially perpendicular to a direction in which the movable pieces are arrayed in a plane containing an optical axis.
Fig. 8 is an explanatory view illustrating movement of the movable piece provided with an SMA actuator.
Fig. 9 is a functional block diagram illustrating an electrical configuration of the photographic device of the present invention.
Fig. 10 is an explanatory view illustrating in schematic form a photographic system of the present invention.
Fig. 11 is a perspective view of a 7x scale model in which eight digital cameras are arranged.
Fig. 12 is a general view of a CMOS camera module.
Fig. 13 is a view of a target organ as photographed by using the camera arrays.
Fig. 14 depicts subject images captured by the first to eighth cameras.
Fig. 15 depicts a stereoscopic image generated from the subject images captured by the third camera C3 and the fourth camera C4.
Fig. 16 depicts a virtual image formed based on a generated three-dimensional model.

### REFERENCE SIGNS LIST

- 2: photographic device
- 4: arm
- 6: first camera array
- 7: second camera array
- 8a-8h: movable pieces
- 10: photographic unit
- 12: illuminant light source
- 15: lens
- 17: image sensor
- 22: hinge (movement control means)
- 32: elastic support
- 35: traction wire
- 37: coil spring
- 40: SMA actuator
- 50: central control unit
- 58: stereoscopic image generation unit (stereoscopic image generation means)
- 60: three-dimensional model generation unit (three-dimensional model generation means)
- 62: communication interface
- 100: image display device
- 102: display screen

### DESCRIPTION OF EMBODIMENTS

### 1. Summary of Invention

The present invention provides a photographic device including a camera array in which plural photographic units are arrayed on a leading end of an arm formed to be capable of passing through a tubular body which is inserted into a body from outside the body, wherein the camera array is displaceably disposed between an alignment location whereat the plural photographic units are arrayed along an extension of the arm and a deployment location whereat the plural photographic units are arrayed laterally to the arm.

In the photographic device of the present invention, displacement between the alignment location and the deployment location is performed by a movement control means for controlling movement of the camera array.

Incidentally, a major cause of limitations of a visual field of a laparoscope arises from a procedure for laparoscopic surgery in itself. Specifically, the visual field of the laparoscope inserted into an abdominal cavity through a trocar (or a tubular body) attached in a small incision made in a body surface is limited to such a visual field as to look around the inside of the abdominal cavity in a sectorial range centered about the trocar. Also, although pneumoperitoneum is performed to provide the largest possible space region in the abdominal cavity, there may be an insufficient distance between an internal organ as an object for surgery and a lens on the leading end of the laparoscope, which in turn may often result only in a narrow field of view.

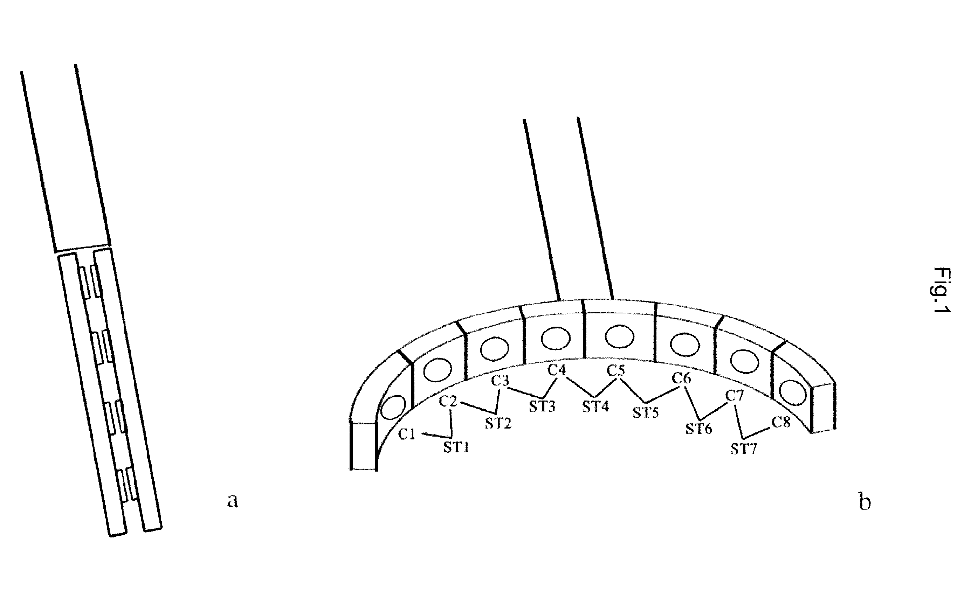

Therefore, the inventor has devised an assembly of plural small-sized video cameras capable of such an arrangement as to surround the internal organ at given angularly spaced intervals on an arc about the target organ. However, it is difficult to insert such an arrangement of multi-viewpoint camera-eyes, as they are, into the abdominal cavity, and thus, the cameras are divided into two left and right arrays as illustrated in Fig. 1a, and a member for fixing the cameras is configured to be flexible, and thereby, the cameras, as transformed as a whole into the shape of a stick like a rigid scope, are inserted into the abdominal cavity through the trocar. A group of the inserted cameras is arranged on the arc in the abdominal cavity for example by pulling a user-operated deployment wire from outside the body (see Fig. 1b). Fig. 1 illustrates eight cameras as mounted. In this case, in order to properly position the cameras on the arc, design is, for example, such that side surfaces of adjacent assemblies mounting the cameras are brought into contact with each other by tension of the wire, the cameras are arranged by setting the positions of the camera arrays with regularity, and the cameras are fixed in the shape of the arc. Also, the deployment wire is loosened to withdraw the cameras from the abdominal cavity, and a folding wire is pulled to restore the cameras to their original shape of the stick.

The photographic device of the present invention is configured as given below; specifically, the shape of the leading end of the photographic device can be transformed into the shape of a long thin stick so that the photographic device can be inserted into the body through the trocar, and, after the insertion of the photographic device into the body, the cameras are deployed in an arcuate manner in the body and are arranged at different positions to photograph images of the inside of the body so that plural images at different viewpoints can be obtained. Image signals are outputted by image sensors, and plural subject images at different viewpoints can be formed based on the image signals from the image sensors. Three-dimensional images (or stereoscopic images) from multiple directions can be simply formed by using two paired subject images among the plural subject images at the different viewpoints.

Description will be given below with regard to more specific forms of the present invention.

### 2. Multi-Viewpoint Camera

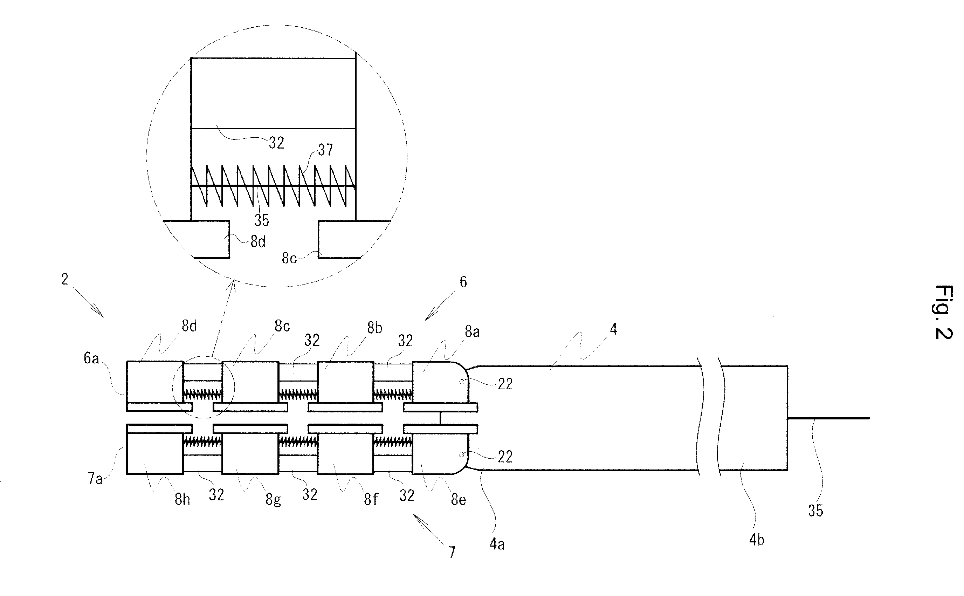

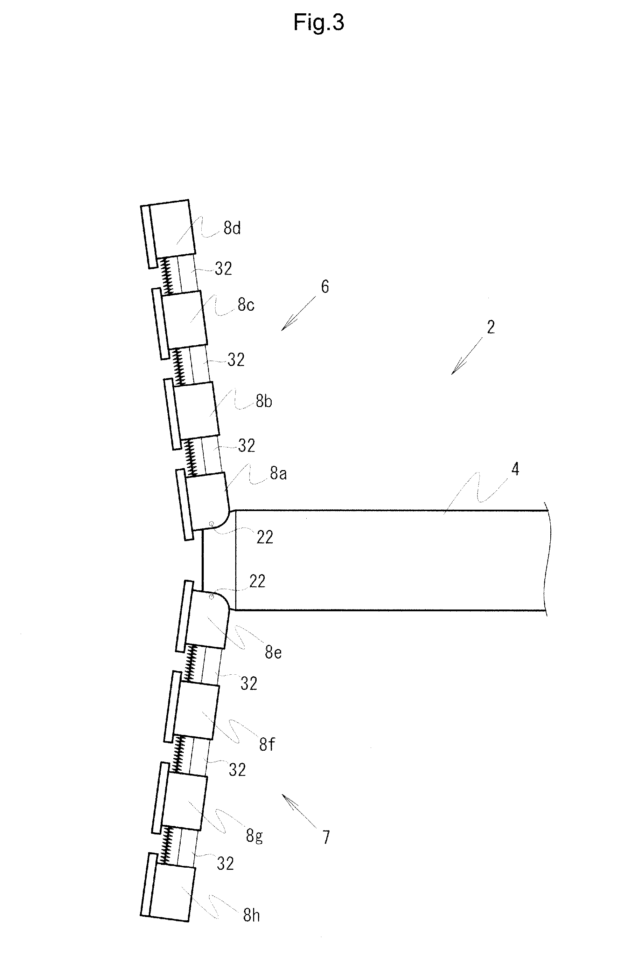

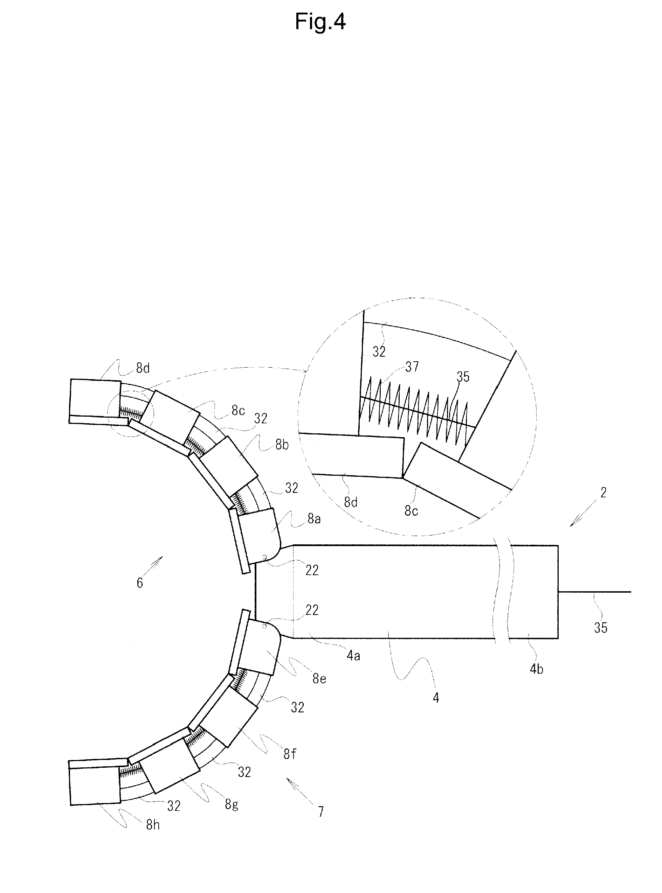

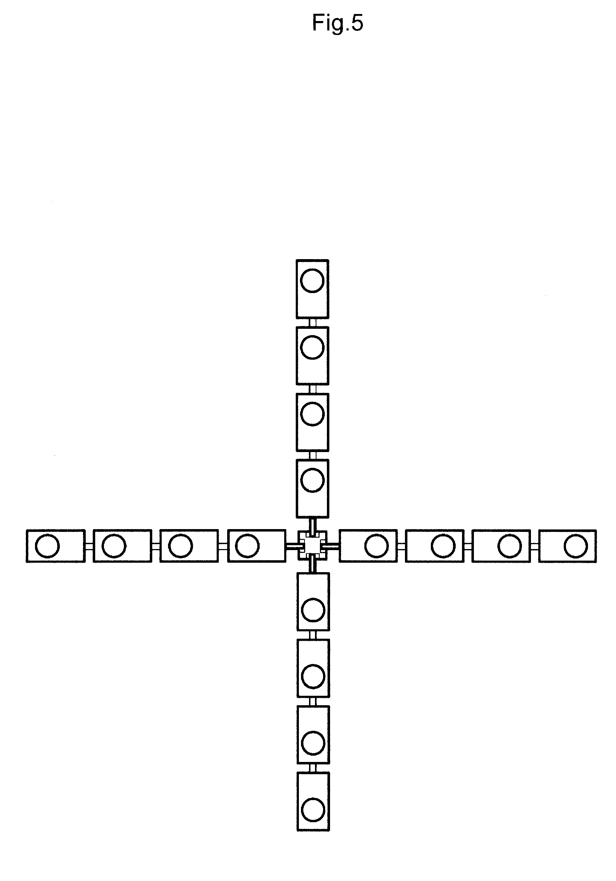

Fig. 2 is a schematic view of assistance in explaining in outline a photographic device having two camera arrays as arranged at an alignment location. Also, Fig. 3 is a view of assistance in explaining the photographic device with the camera arrays as deployed outward, and Fig. 4 is a view of assistance in explaining the photographic device with the outwardly deployed camera arrays as bent and curved in an arcuate manner. Fig. 5 is a view of four camera arrays as deployed.

As illustrated in Figs. 2 to 4, a photographic device 2 of the present invention includes an arm 4 and first and second camera arrays 6, 7, and the first and second camera arrays 6, 7 are arranged on the leading end of the arm 4. Each of the camera arrays 6, 7 has plural movable pieces 8; the first camera array 6 has first to fourth movable pieces 8a to 8d, and the second camera array 7 has fifth to eighth movable pieces 8e to 8h. The arm 4 is formed to be long and thin, and the leading end of the arm 4 is provided, on hinges 22, with the camera arrays 6, 7 formed to be long and thin. The arm 4 and the camera arrays 6, 7 are formed to be long and thin, and thereby, the photographic device 2 can be introduced into a patient's body through a tubular body such as a trocar inserted into the patient. Also, the number of camera arrays is not limited to two, and three or more camera arrays may be provided. Fig. 5 is a view of four camera arrays as deployed. The camera arrays may be deployed in a straight line as illustrated in Fig. 3, or may be deployed in a shape such that the arrays are located along a hemispherical surface (for example, in a shape like rib portions of an umbrella as unfolded), by being bent and curved in an arcuate manner as illustrated in Fig. 4.

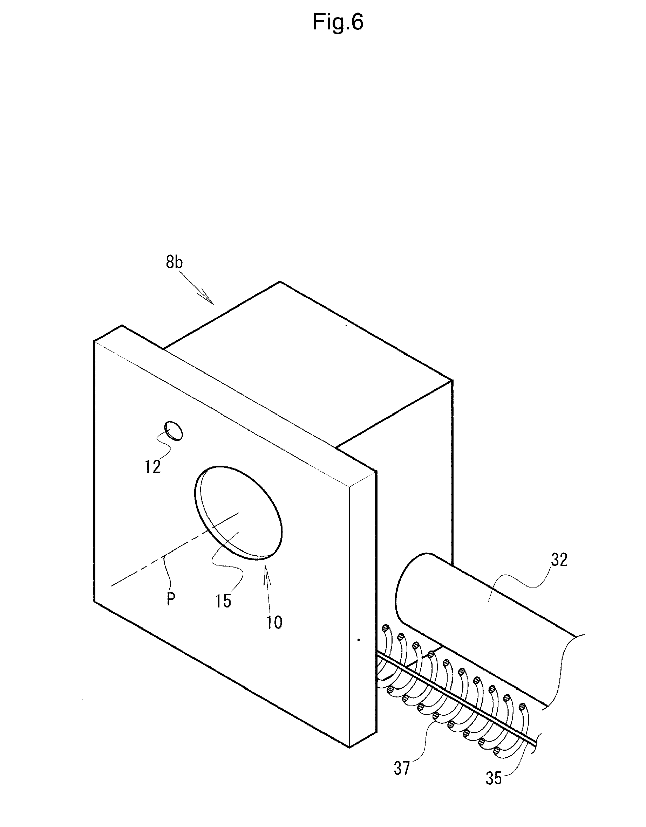

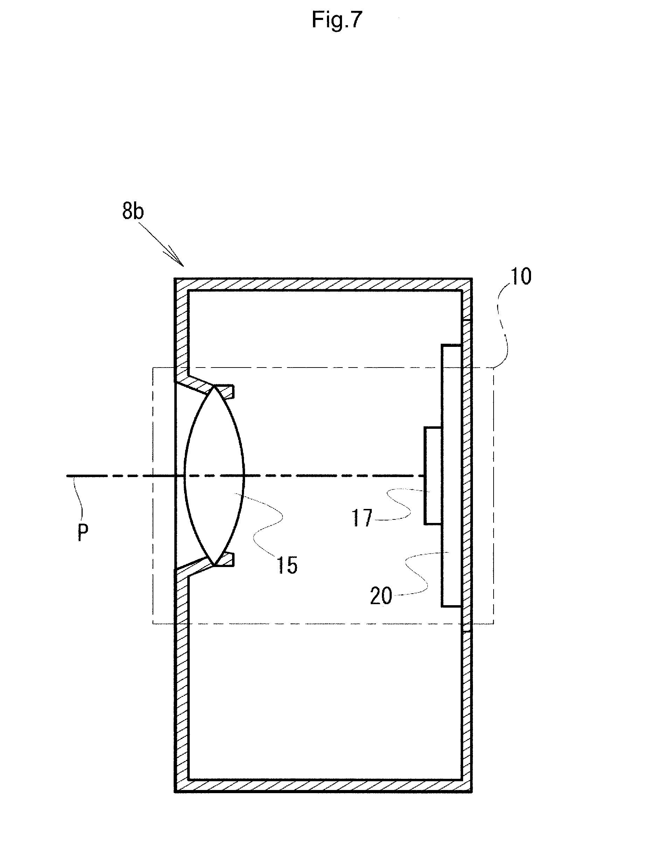

Fig. 6 is a view of assistance in explaining each of the movable pieces which forms each of the camera arrays 6, 7, and a photographic unit and the like included in each of the movable pieces. Also, Fig. 7 is a cross-sectional view of the movable piece taken in a direction substantially perpendicular to a direction in which the movable pieces are arrayed in a plane containing an optical axis. Here, the movable piece 8b is illustrated by way of example. As illustrated in Figs. 6 and 7, each of the movable pieces 8a to 8h includes a photographic unit 10 and an illuminant light source 12. The photographic unit 10 includes a lens 15 facing a subject, an image sensor 17 located on the image surface side of the lens 15, and a circuit board 20 provided with a signal processing circuit which amplifies and digitizes a signal from the image sensor 17 to form image data. A CMOS (complementary metal oxide semiconductor) image sensor or a CCD (charge coupled device) image sensor, for example, may be used as the image sensor 17, and the use of the CMOS type image sensor, in particular, enables reducing power consumption or an external size. Also, it is preferable that an LED (light emitting diode) light source be used as the illuminant light source 12, and the use of the LED light source enables making the movable piece compact and achieving high luminance and low power consumption. Incidentally, although unillustrated, a shutter device, an iris, or a variable magnification device may be built in the photographic unit 10, as needed. The photographic unit having these devices built-in enables obtaining an image of higher quality.

The camera arrays 6, 7 are rotatably mounted to the arm 4 for example by the hinges 22 as movement control means (see Fig. 2). The camera arrays 6, 7 mounted to the arm 4 by the hinges 22 can move between an alignment location (see Fig. 2) at which the photographic units 10 are arranged along an extension of the arm 4 with free ends 6a, 7a located along the extension of the arm 4 and a deployment location (see Figs. 3 and 4) at which the photographic units 10 are arranged extending laterally to the arm 4 with the free ends 6a, 7a of the camera arrays 6, 7 located on both sides of the arm 4 with the hinges 22 in between. Although user operation may control movement of the camera arrays 6, 7 between the alignment location and the deployment location or various motors such as a servomotor may be used to control the movement, the camera arrays 6, 7 are here structured so that the user pulls a traction wire 35 to allow the camera arrays 6, 7 to move from the alignment location to the deployment location and the user loosens the traction wire to allow the camera arrays 6, 7 to move from the deployment location back to the alignment location. When the camera arrays 6, 7 are located at the deployment location, the lenses 15 included in the movable pieces 8a to 8h are exposed.

Preferably, in each of the camera arrays 6, 7, the photographic units 10 of the movable pieces 8 are arrayed at substantially equally spaced intervals, extending from the fixed end side of the camera array 6 toward the free end side thereof. The illuminant light source 12 is disposed in the vicinity of the lens 15 (see Fig. 6), and thereby, the dark inside of the body can be brightly lit up for photographing. A distance between the adjacent lenses 15 between their optical axes, although not particularly limited, is set preferably within a range of 5 to 100 mm or more preferably within a range of 10 to 70 mm. Setting such a distance enables obtaining the same three-dimensional image as that when observed with human eyes. This can be achieved by one of the adjacent photographic units 10 capturing a subject image for the left eye, and by the other photographic unit 10 capturing a subject image for the right eye. Also, even if the photographic units are not adjacent to each other, subject images captured by the first photographic unit from the arm 4 side (or the photographic unit mounted on the movable piece 8a or 8e) and the third photographic unit therefrom (or the photographic unit mounted on the movable piece 8c or 8g), starting counting at the arm 4 side, for example, may be used as an image for the left eye and an image for the right eye, respectively, thereby to form stereoscopic image data rich in desirable three-dimensional appearance from free viewpoints.

The movable pieces 8a to 8h can be connected for example by elastic supports 32 and coil springs 37. The elastic support 32 is formed in the shape of a hollow tube, and has a harness or a cable passed internally therethrough, which extends from the circuit board 20 to be described later (see Fig. 7), included in each of the movable pieces 8a to 8h, toward the arm 4. The coil spring 37 interposed in between the movable pieces is fixed at its ends to the movable pieces, respectively, and is configured to apply a bias force according to movement of the movable pieces.

As illustrated in Figs. 2 and 6, the biasing coil spring 37 having the traction wire 35 passed therethrough can be disposed on the subject side between the movable pieces 8a to 8h, and the flexibly bendable elastic support 32 can be disposed on the image side therebetween. One end of the traction wire 35 is exposed from an end portion of the arm 4 so that the user can pull the traction wire 35, and the traction wire 35 is passed through the movable pieces 8a to 8c and 8e to 8g and is fixed at the other end to the movable pieces 8d and 8h. The spring 37 is disposed between the movable pieces, and the traction wire 35 is passed internally through the spring 37. The following is effected by this configuration; specifically, when the user pulls the one end of the traction wire 35, the movable pieces 8d, 8h fixed to the other end of the traction wire 35 are pulled against the bias force of the spring 37, and thus, a distance between the adjacent movable pieces on the subject side becomes shorter than a distance therebetween on the image surface side, and thereby, the camera arrays 6, 7 are transformed into an arcuate shape as illustrated in Fig. 4. At this time, the optical axes of the lenses 15 of the movable pieces 8a to 8h are oriented toward a predetermined position inwardly of the arc. Thereby, images of a target object in the subject can be photographed from different angles. As described more specifically (see Fig. 4), the second movable pieces 8b, 8f located adjacent to the movable pieces 8a, 8e, respectively, mounted rotatably to the arm 4 by the hinges 22 are supported at their image side by the flexible elastic supports 32, and thus, the movable pieces 8b, 8f are displaced so that their respective distances to the movable pieces 8a, 8e on the subject side become less than those on the image side. As a result, the second movable pieces 8b, 8f are displaced so that the optical axes of the lenses 15 included in the movable pieces 8b, 8f are oriented inward. Likewise, the third movable pieces 8c, 8g from the movable pieces 8a, 8e, respectively, starting counting at the movable pieces 8a, 8e, are also mounted to the movable pieces 8b, 8f, respectively, by the elastic supports 32 and the biasing coil springs 37, and thus, the movable pieces 8c, 8g are displaced so that the lenses 15 included in the movable pieces 8c, 8g are oriented more inward than those of the movable pieces 8b, 8f. Also, likewise, the movable pieces 8d, 8h can be displaced so that the lenses 15 of the fourth movable pieces 8d, 8h from the movable pieces 8a, 8e, respectively, starting counting at the movable pieces 8a, 8e, are oriented more inward than those of the movable pieces 8c, 8g.

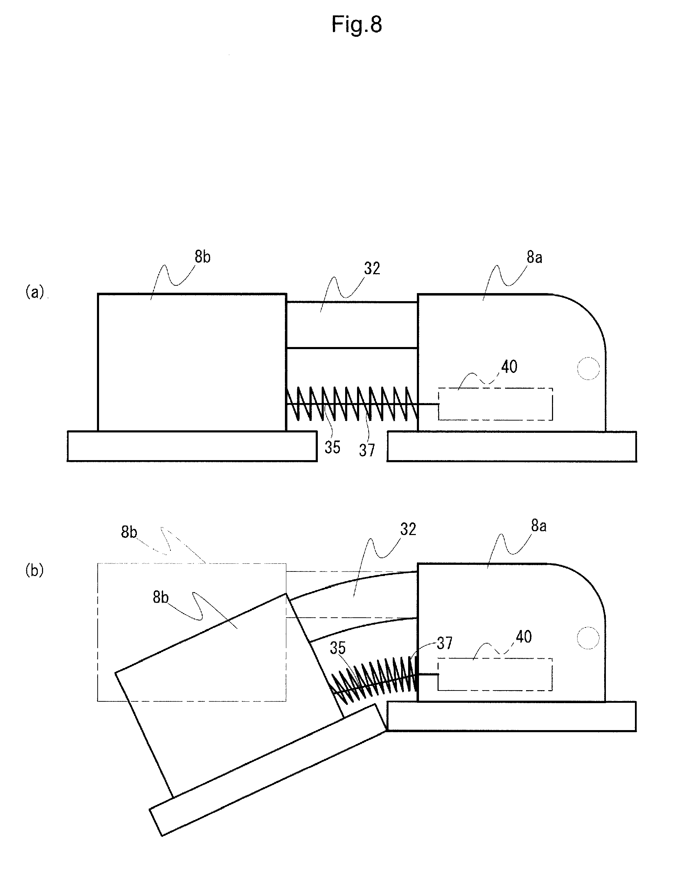

In the above description, the user directly pulls and operates the traction wire 35 to transform the camera arrays 6, 7 into the arcuate shape; however, an SMA (shape memory alloy) actuator may be used to transform the camera arrays 6, 7 into the arcuate shape. Fig. 8 is a view of assistance in explaining movement of the second movable piece 8b displaceably supported by the first movable piece 8a with the support in between. A portion between the movable piece 8a and the movable piece 8b is illustrated by way of example. As illustrated in Fig. 8(a), for example, one end of the traction wire 35 is connected to an SMA coil spring 40 provided in the movable piece 8a, and the other end of the traction wire 35 is fixed to the movable piece 8b. When a current is passed through the SMA coil spring 40, as illustrated in Fig. 8(b), a distance between the movable piece 8a and the movable piece 8b on the subject side becomes shorter than a distance therebetween on the image side, and the subject side of the movable piece 8b is drawn close to the movable piece 8a. Such a mechanism may also be used in the movable pieces 8c to 8h to transform the camera arrays 6, 7 into the arcuate shape. A generally known SMA actuator may be used as the SMA actuator 40; for example, the SMA actuator is formed in a coiled fashion, and the coil shrinks when the SMA actuator reaches its transformation temperature or higher by the passage of a current through the SMA actuator. Thus, the SMA actuator may also be used to transform the camera arrays into the arcuate shape.

When the pulling of the traction wire 35 by the user is released, the bias force of the biasing coil spring 37 becomes greater than a tensile force of the traction wire 35, and the movable pieces located on both sides of the biasing coil spring 37 are separated from each other by the bias force of the biasing coil spring 37 having the traction wire 35 passed therethrough, so that the camera arrays 6, 7 are restored to the alignment location as illustrated in Fig. 2. Thereby, optical axes P (see Fig. 7) of the lenses of the adjacent movable pieces 8a to 8d and 8e to 8h are restored to their substantially parallel state. The camera arrays 6, 7 are moved from the deployment location to the alignment location, and thereby, the camera arrays 6, 7 can be transformed into the shape of a stick and be pulled out of the body through the trocar.

In the above description, the first movable pieces 8a, 8e are rotatably mounted to the arm 4 with the hinges 22 in between; however, as is the case with the disposition of the hinges between the arm and the movable pieces, SMA actuators may be used in place of the hinges to effect the movement of the camera arrays between the alignment location and the deployment location. The SMA actuators are used to effect movement of the movable pieces between the alignment location and the deployment location, and thereby, the photographic device, with the camera arrays closed in the shape of the stick, can be inserted into the body through the trocar, and the camera arrays, after inserted into the body, can be moved to the deployment location to capture plural subject images at different viewpoints. In this case, the positions of the camera arrays located at the deployment location are measured beforehand, and a distance between the lenses, an angle of congestion or a base length formed by the optical axes of the lenses included in the adjacent photographic units, or the like is stored beforehand as data in ROM (read only memory), thereby improving convenience.

### 3. Photographic Device

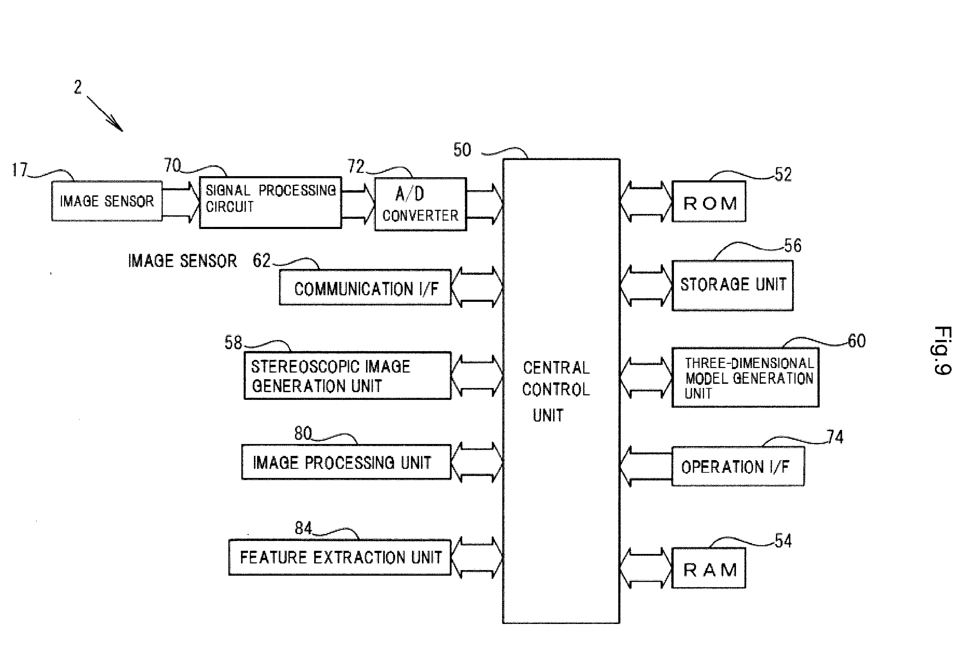

Fig. 9 is a functional block diagram illustrating in schematic form an electrical configuration of the photographic device of the present invention. As illustrated in the block diagram of Fig. 9, the photographic device 2 includes a central control unit 50, ROM 52, RAM (random access memory) 54, a storage unit 56, a stereoscopic image generation unit 58 (or a stereoscopic image generation means), a three-dimensional model generation unit 60 (or a three-dimensional model generation means), a communication interface 62 (hereinafter abbreviated as a communication I/F), a signal processing circuit 70, an A-D (analog-to-digital) converter 72, an operation interface 74 (hereinafter called an operation I/F), an image processing unit 80, a feature extraction unit 84, and the like. Various programs for use in the photographic device 2 are stored beforehand in the ROM 52, and, at the time of use of the programs, the programs are loaded into the RAM 54 for their use. The storage unit 56 accesses a recording medium or the like to record image data or do the like.

An optical image of a subject from the lens 15 is formed on a photo-receptive area of the image sensor 17 such as a CMOS image sensor, and an image signal from the image sensor 17 is fed to the signal processing circuit 70. The signal processing circuit 70 includes an amplifier which amplifies the image signal, and a gain correction circuit which corrects the amplified image signal, thereby to amplify and correct the image signal. The image signal amplified and corrected by the signal processing circuit 70 is converted by the A-D converter 72 from an analog signal to a digital signal, which is then fed as image data into the image processing unit 80. The image data inputted to the image processing unit 80 is corrected for contour, white balance, brightness, contrast, or the like, and is stored in the storage unit 56.

The image data stored in the storage unit 56 is put in order and stored for example for each photographing, and plural subject images at different viewpoints captured by the first to eighth photographic units 10 for each photographing are collectively grouped and stored. In the case of storage of a grouping of the image data, a folder is created for each photographing, and eight images photographed by the first to eighth photographic units 10, for example, are stored in the folder, and thereby, the image data can be put in order and stored for each photographing.

The stereoscopic image generation unit 58 forms stereoscopic image data capable of stereoscopic display of the images of the subject, for example by using subject image data from the two adjacent photographic units 10, among the plural subject images at the different viewpoints grouped and stored in the storage unit 56 for each photographing. A display of the subject having a three-dimensional appearance can be provided for the user, by displaying a subject image for the right eye and a subject image for the left eye on an image display device to be described later, by using one of two pieces of subject image data, for example the subject image captured by the photographic unit 10 located on the left, as the image for the left eye, and using the other subject image data, for example the subject image captured by the photographic unit 10 located on the right, as the image for the right eye.

When it is desired to provide a display of an image richer in three-dimensional appearance for the user, a display of a subject image richer in three-dimensional appearance can be provided for the user by forming stereoscopic image data for example by using subject image data captured by the photographic unit 10 of the first movable piece 8a and the photographic unit 10 of the third movable piece 8c so as to produce greater parallax.

The three-dimensional model generation unit 60 generates a three-dimensional model (or three-dimensional geometric data) based on image data from the photographic units 10. Although there are various methods for forming the three-dimensional model, a stereoscopic method, a visual volume intersection method, and a factorization method, for example, have heretofore been well known, and any of these methods may be used to generate the three-dimensional model. For example in the stereoscopic method, the feature extraction unit 84 extracts features on each of plural subject images at different viewpoints. A heretofore known procedure may be used as a procedure for feature extraction, and brightness, contrast, contour or other information may be used as appropriate for calculation. A point corresponding to each of feature points extracted by the feature extraction unit is determined across the images, and a distance to the point is determined by using the principles of triangulation. This can be accomplished in the following manner; specifically, position information on the photographic units 10 on the camera arrays 6, 7 is stored beforehand in the ROM 52, and a distance to a feature point of a target object can be determined from the position information and angle information on the feature point on the target object in the subject, and thereby, the three-dimensional model of the target object can be generated. This enables achieving a grasp of a stereostructure of the overall surgical field, and a grasp of the absolute position of a surgical instrument with respect to the overall surgical field.

Besides the stereoscopic method, the visual volume intersection method may also be used. A visual volume is a pyramid having a viewpoint as a vertex and having a silhouette of a target object as a cross section, and the visual volume intersection method involves determining a common portion of the visual volumes of the target object at all viewpoints, thereby generating a model of the target object. Thus, the silhouettes of the target object are extracted from images photographed by plural photographic units arranged at different positions, and an intersection of the silhouettes is calculated. The factorization method may be used as a method for generating the three-dimensional model, other than the above-described stereoscopic method and visual volume intersection method. The generated three-dimensional model is stored in the storage unit 56.

By using the three-dimensional model, the user can freely change viewpoints to observe the target object contained in the subject; thus, for example, when it is desired to closely observe the target object from various virtual viewpoints for surgery or the like, the viewpoints may be arbitrarily set to form images based on the three-dimensional model and display the images on the image display device to be described later.

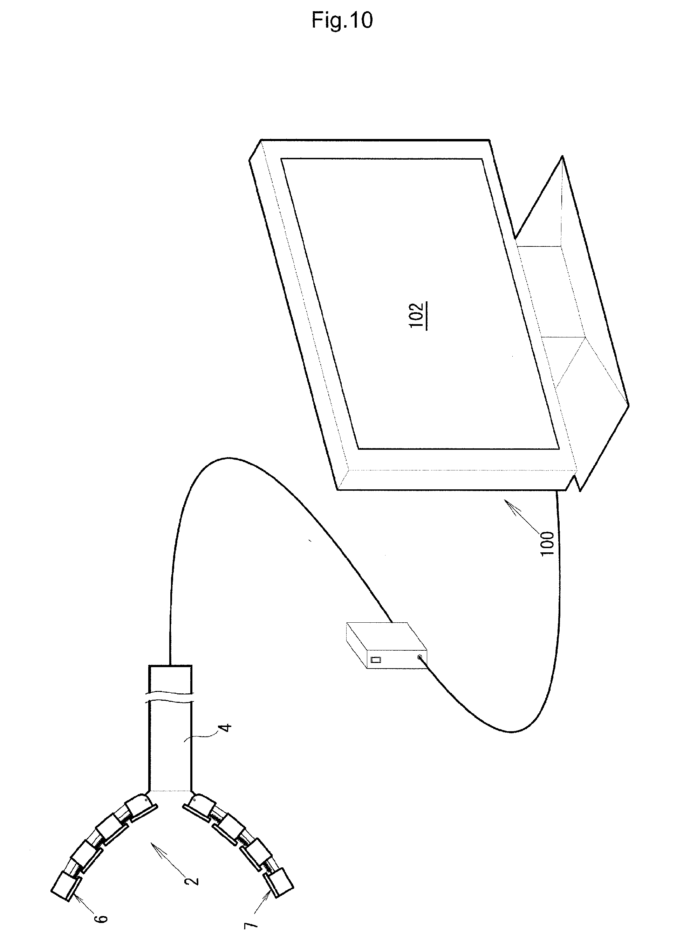

The communication I/F 62 transmits plural pieces of image data at different viewpoints, the stereoscopic image data, and the three-dimensional model (or three-dimensional geometric data), stored in the storage unit 56, to an external device. For data transmission to the external device, the data is modulated to form a radio signal, which is then transmitted from an antenna (unillustrated). The radio signal transmitted from the communication I/F 62 is received for example by the image display device illustrated in Fig. 10.

### 4. Image Display System

Fig. 10 is a schematic view illustrating a configuration of an image display system including the photographic device of the present invention and the image display device communicably connected to the photographic device. The image display system is formed by the photographic device 2 and an image display device 100, and the photographic device 2 and the image display device 100 are connected so as to be capable of information transmission. Besides a display placed on a desk, anything, such as a personal computer provided with a display, portable electronic equipment provided with a display screen, or a head-mounted display put on the user's head and provided with a small-sized liquid crystal display panel for the left eye and a small-sized liquid crystal display panel for the right eye, may be used as the image display device 100, provided only that it can communicate with the photographic device to display an image.

The image display device 100 includes an image display surface 102, a display control unit (unillustrated), and a communication I/F, and the display control unit controls an image displayed on the image display surface 102. Forms of image display are as follows; for example, the display control unit may display plural subject images at different viewpoints in side-by-side arranged or partially overlapping relation on the image display surface 102, or may also display in enlarged dimension a subject image selected from among the subject images by the user.

Further, the target object in the subject (for example, an internal organ in the body, such as the liver) is displayed based on the three-dimensional model, and an image based on the three-dimensional model can freely vary in viewpoint.

Also, an image for the right eye and an image for the left eye may be displayed side by side on the image display surface 102, based on the stereoscopic image data, thereby to provide a display of a three-dimensional image of the subject for the user. When the head-mounted display is used, a stereoscopic image can be provided for the user by displaying the image for the left eye on the display panel for the left eye, and displaying the image for the right eye on the display panel for the right eye.

Next, description will be given with regard to operational advantages of the present invention. The first to eighth movable pieces 8a to 8h are transformed into the shape of a stick with the fifth to eighth movable pieces 8e to 8h facing the first to fourth movable pieces 8a to 8d, in order that the leading end of the photographic device 2 is inserted into the patient's body for example through the trocar inserted into the body from outside the body. Thereby, the first to eighth movable pieces 8a to 8h can be inserted into the body through the trocar. After the insertion of the first to eighth movable pieces 8a to 8h into the body, the first and fifth movable pieces 8a, 8e swing about the arm 4, and correspondingly, the second to fourth movable pieces 8b to 8d and the sixth to eighth movable pieces 8f to 8h coupled to the first and fifth movable pieces 8a, 8e, respectively, are displaced so as to be separated from each other.

When the SMA actuators 40 included in the movable pieces operate, the traction wire 35 is pulled and the lenses 15 included in the first to eighth movable pieces 8a to 8h are oriented toward a predetermined location. The SMA actuators 40 of the first to eighth movable pieces 8a to 8h operate to orient the lenses 15 toward a location preset for the camera arrays 6, 7, thereby enabling the photographing of the subject. When the photographic units 10 included in the first to eighth movable pieces 8a to 8h are used to photograph the subject, eight subject images at different viewpoints, for example, can be captured.

Stereoscopic image data for stereoscopic display can be formed based on image data from the image sensors 17 provided in the adjacent movable pieces, among the eight subject images at the different viewpoints. Also, a three-dimensional model for the target object in the subject can be generated based on plural images at different viewpoints (e.g. up to eight images in Figs. 2 to 4). By generating the three-dimensional model of the target object, a virtual image of the target object as observed from any user-specified viewpoint, for example, can be displayed on the image display screen 102 of the display device 100.

At the time of pulling of the photographic device 2 out of the body, the first to eighth movable pieces 8a to 8h located at the deployment location are displaced to their closed position thereby to transform the leading end of the device into the shape of a stick. At the time of shift of the first to eighth movable pieces 8a to 8h to the closed position, a current passing through the SMA actuators 40 is cut off to transform the camera arrays 6, 7 into the form of a straight line, and the camera arrays 6, 7 are faced with each other so that the lenses 15 of the first and fifth movable pieces 8a, 8e face each other, and thereby, the second to fourth and sixth to eighth movable pieces can be arrayed along the extension of the arm 4, and the first and second camera arrays 6, 7 of the photographic device 2 can be changed into the shape of a stick. Thus, the camera arrays 6, 7 are shifted from the open deployment location to the closed alignment location, and thereby, the leading end portion of the photographic device 2 can be pulled out of the body through the trocar.

Also, in the above description, a stereoscopic image and a three-dimensional model are generated on the part of the photographic device 2, and an image based on the stereoscopic image or the three-dimensional model is displayed on the display screen 102 of the image display device 100 communicably connected to the photographic device 2; however, the generation of the stereoscopic image or the generation of the three-dimensional model is not performed by the photographic device 2 but may be implemented on the part of the image display device. For example, when a computer device having an image processing circuit is used as the image display device, plural subject images at different viewpoints captured by the photographic device are transmitted to the computer device, and the computer device forms a stereoscopic image or a three-dimensional model, based on the received images, and displays the formed image on the display screen. The generation of the stereoscopic image or the three-dimensional model requires relatively high computing power, and thus, the photographic device of the present invention is connected to the computer device having high computing power, and thereby, the image based on the plural subject images, the stereoscopic image or the three-dimensional model can be more smoothly displayed.

Also, in the above-described embodiment, the camera arrays 6, 7 are transformed into the arcuate shape; however, this mechanism may be omitted so that the device becomes simpler. For example, when the camera arrays are in their position illustrated in Fig. 3, the orientations of the photographic units 10 may be preset so that the lenses are oriented toward a predetermined location, and thereby, plural subject images can be captured without the camera arrays being transformed into the arcuate shape.

According to the photographic device and the image display system of the present invention, as described above, the device and the system include a camera array disposed rotatably about one end, on the leading end of an arm (or a supporting member) which is inserted into a body; plural cameras arrayed on the camera array at predetermined spaced intervals, extending from the fixed end side to the free end side of the camera array, and oriented toward a location preset for the camera array; and a movement control means for controlling movement of the camera array between an alignment location at which the plural cameras are arrayed along an extension of the arm with the free end of the camera array located along the extension of the arm and a deployment location at which the plural cameras are arrayed laterally to the arm with the camera array swung from the alignment location. Thereby, the camera array is located at the alignment location and thereby the camera array is inserted into the body through the tubular body such as the trocar, and the inserted camera array is moved to the deployment location and thereby plural subject images at different viewpoints can be captured by using the plural cameras. The capture of the plural subject images at the different viewpoints enables generating a stereoscopic image or generating a three-dimensional model. A display of the generated stereoscopic images as an image for the left eye and an image for the right eye, for example, is provided for the user, and thereby, a display of a subject image rich in three-dimensional appearance can be provided for the user. Also, an image of a target object contained in the subject, as observed from any user-selected viewpoint, can be displayed based on the generated three-dimensional model, and thus, it is possible to provide a system having a high degree of convenience, which can accurately display a portion of a desired target object to be observed by the user.

### EXAMPLES

The inventor has prepared and verified the photographic device of the present invention.

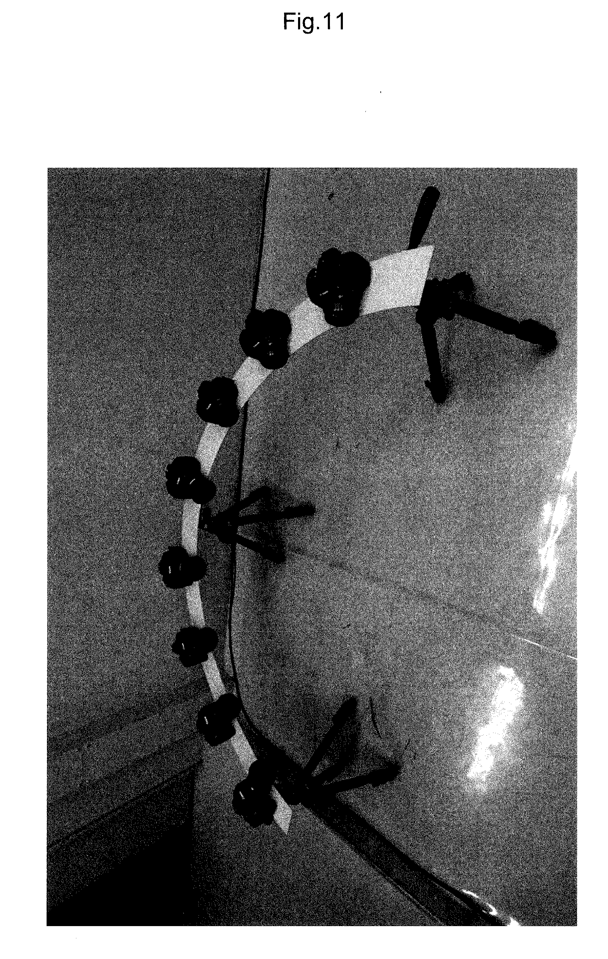

In a development phase, first, in order to obtain the required number of viewpoints of cameras for the inside of the abdominal cavity, an angle of arrangement, and a proper visual field angle of each camera, typical digital cameras were used to prototype a model of a 7x scale size as depicted in Fig. 11, and a structure of a multi-viewpoint camera system to be designed was determined. The system uses eight digital cameras, namely, first to eighth cameras C1 to C8, and is the 7x scale model in which the digital cameras are arranged at equally spaced intervals on a semicircular camera mount having a radius of 65 cm, and the devised system was examined for basic performance. Thereby, eight subject images at different viewpoints can be captured, and further, pairs of images captured by the adjacent cameras can be obtained; specifically, seven stereoscopic images ST1 to ST7 can be captured by using the eight cameras, where, for example, ST1 indicates a pair of subject images (hereinafter called a stereoscopic image) captured by the camera C1 and the camera C2, and likewise, ST2 indicates a stereoscopic image captured by the camera C2 and the camera C3.

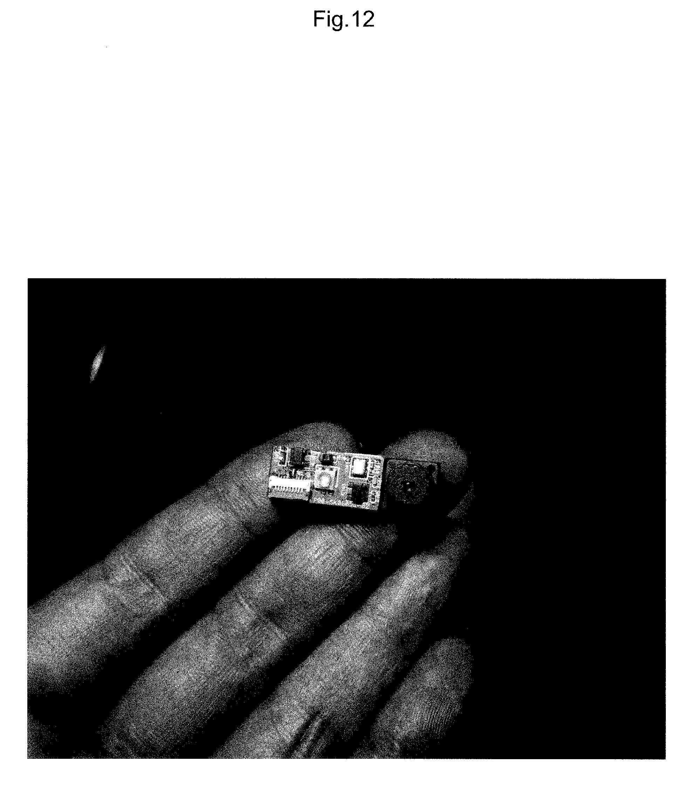

Based on this prototype, a prototype multi-viewpoint camera in which cameras are transformed from the shape of a stick (see Fig. 1a) into an arrangement of the cameras on an arc having a radius of 7 cm for example (see Fig. 1b), as illustrated in Fig. 1, was prepared by using eight CMOS video cameras with a resolution of 1024-by-768 as depicted in Fig. 12, and experiments using a removed organ were performed to verify viewpoint movement capability, stereoscopy capability, and four-dimensional display capability, as given below.

Electric circuits for driving the CMOS video cameras were collectively arranged on boards having dimensions of 15 mm by 10 mm. Light sources for the cameras were also arranged on the boards so that a target organ could be uniformly illuminated. Also, flexible cables located behind camera assemblies were used as power supply lines and signal lines to the boards thereby to reduce the amount of cables and hence achieve miniaturization of the overall device.

### Preparation of Image Display Capability

### Acquisition of Free Viewpoint

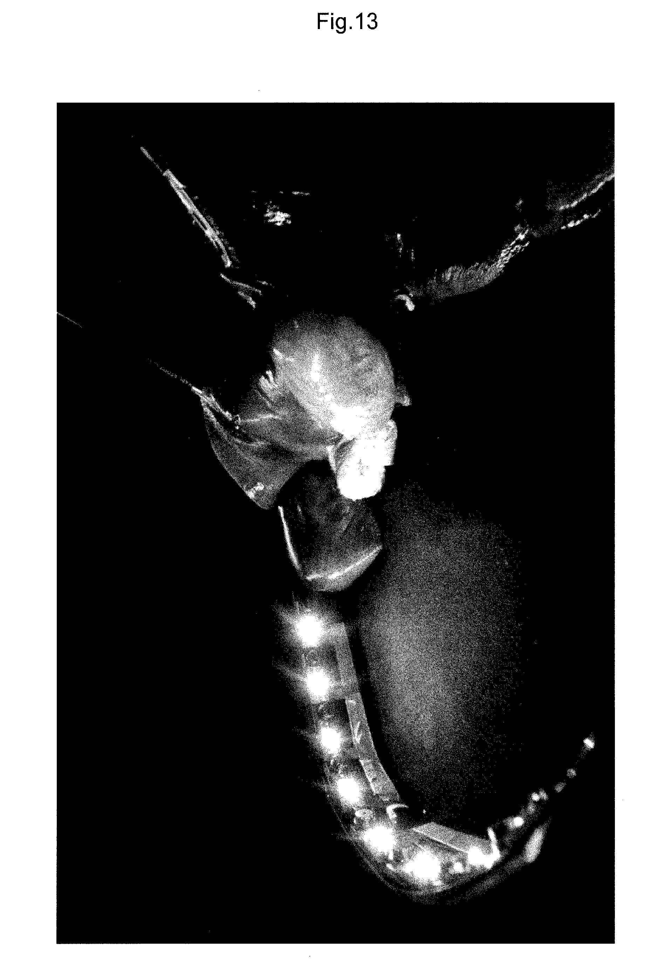

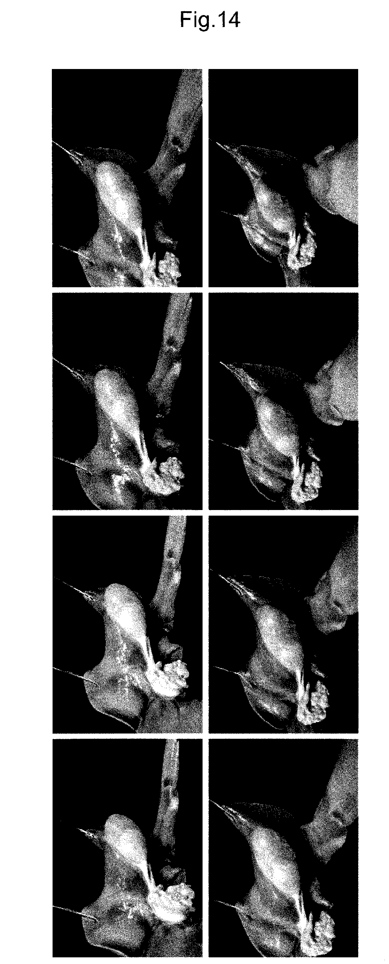

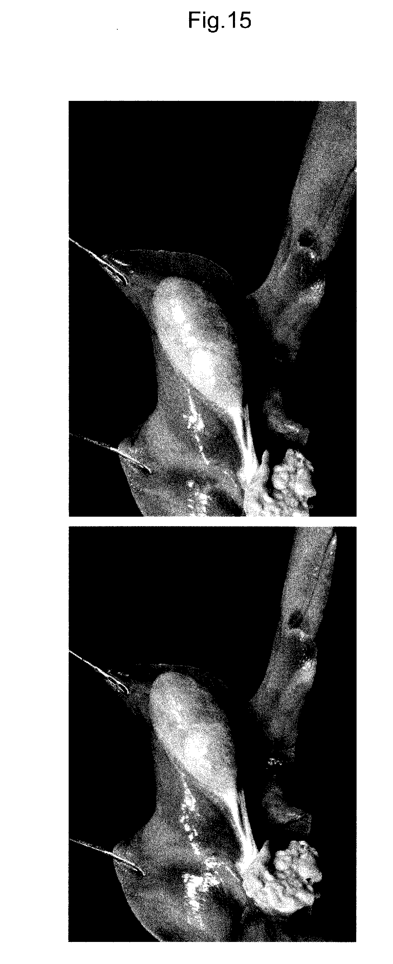

Eight subject images at different viewpoints could be simultaneously captured as depicted in Fig. 14, by packaging the capability of varying visual fields without physical movement of the cameras in the abdominal cavity, by freely selecting viewpoints of the eight video cameras arranged on the arc with respect to a target object in the abdominal cavity, as depicted in Fig. 13. Also, the cameras operate independently of one another, and thus, a function which enables a person who performs surgery or the like to observe the inside of the abdominal cavity from other directions by plural monitors was also provided as needed. Further, the viewpoints of the adjacent cameras were utilized to enable stereoscopy (see Fig. 15) and thus enable arbitrarily selecting stereoscopic images from eight directions (ST1 to ST7). Fig. 15 depicts details of the stereoscopic image ST3 generated from subject images captured by the third camera C3 and the fourth camera C4 illustrated in Fig. 1b.

### Grasp of Time-Varying Stereostructure of Abdominal Cavity

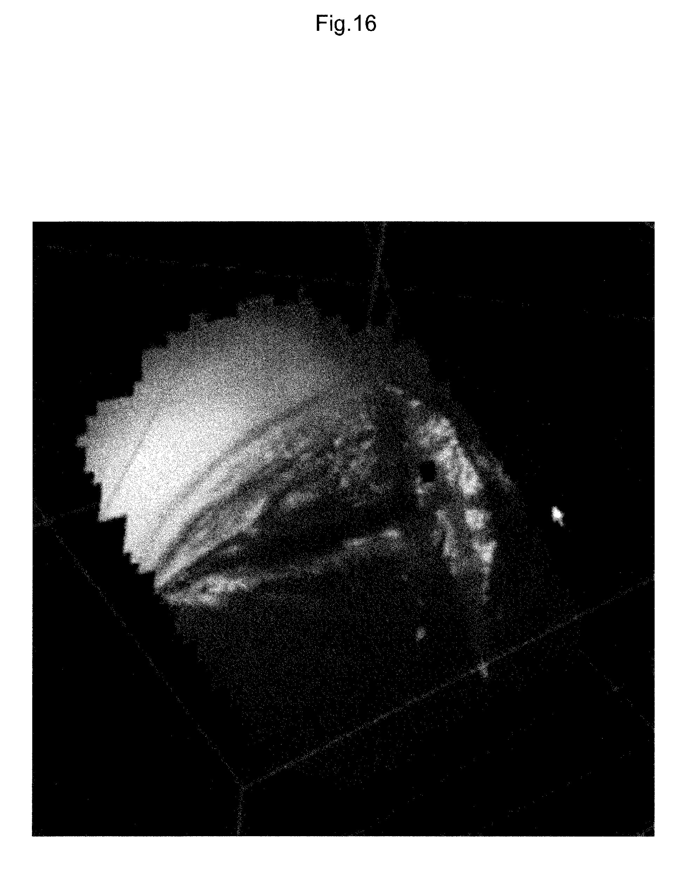

Also, as depicted in Fig. 16, there was packaged the capability of measuring a surface shape of the organ and generating a three-dimensional model by using the images from the eight directions, and displaying the model as a three-dimensional geometry, and, further, mapping the color and texture of this portion. This enabled achieving a grasp of changes, with time, in a three-dimensional shape of the target organ (or the target object) which varies by incision, cauterization, excision or the like as surgery proceeds.

### Results

### Multi-Viewpoint Camera

### Acquisition of Free Viewpoint and Acquisition of Stereoscopic Image

Fig. 15 depicts images at viewpoints from eight directions obtained by an experiment using the extirpated liver and gallbladder, and the positions of the images correspond to camera numbers, respectively, illustrated in Fig. 1b. As a result, 30 frames of images per second could be captured at a resolution of 1024-by-768 from eight viewpoints on the arc having a radius of 7 cm and a central angle of 160°. The captured images were displayed on a main monitor by selecting arbitrary viewpoints by a control unit outside the body, and this enabled observing the state of an internal organ in a surgical part from many viewpoints without physical movement of the cameras in the body. Also, it has been shown that the control unit can output the images captured by all the eight cameras to display and use the images on an auxiliary monitor group, as needed.

### Acquisition of Four-Dimensional Information on Surgical Region

Images from eight directions as depicted in Fig. 14, at arbitrary time, were captured, the surface shape of the organ in a visual field at that time was measured for surface modeling, and colors and textures obtained from the video images were added, thereby enabling a grasp of a stereostructure of a target part for surgery at that time. As depicted in Fig. 16, the created stereostructure was displayed on the auxiliary monitor so that the stereostructure could be interactively enlarged, reduced and rotated. It has been shown that, on the obtained three-dimensional images, not only the surface shape of the target organ but also the position and shape of a surgical instrument located near the organ can be grasped.

For the present invention, the capabilities were examined by using the 7x model of an actual development machine, and this data was used to develop a test machine of a size which can be used in the abdominal cavity. Then, in vitro experiments were performed by using livers and gallbladders removed from pigs, information possessed by obtained images was checked, and its usefulness could be examined. As a result, it has been shown that the acquisition of free viewpoints without entailing physical movement of the cameras yields a new viewpoint for laparoscopic surgery. Also, it has been shown that, the acquisition of vision having depth direction information from a stereoscopic image capable of viewpoint movement enables safer laparoscopic surgery.

Also, it has been shown that the surface shape of the organ obtained from the captured images from the eight directions has the capability of grasping three-dimensional changes in a surgical field on the time series, and it has been shown that the position or volume of a part to be excised or the like can also be quantitatively measured.

Also, based on information on the surface shape of the organ, a chest image of blood vessels, a tumor or the like in the target organ obtained by X-ray CT (computed tomography) or MRI (magnetic resonance imaging) before surgery is superposed and displayed on a captured surgical field image, and thereby, in laparoscopic surgery or robotic surgery, more diversified information can be provided to a person who performs surgery.

Further, information on four-dimensional changes in a surgical field can be utilized by providing the capability of capturing images at certain time intervals and constructing the surface shape, and also, keeping track of the constructed three-dimensional image and multi-viewpoint image information on the time series can serve as a means for quantitatively verifying accidental occurrence of malpractice, the degree of skill of a person who performs surgery, or the like.

### INDUSTRIAL APPLICABILITY

The present invention provides a photographic device and a photographic system capable of photographing the inside of a body.

According to the photographic device of the present invention, it is possible to provide a camera system for rigid scope surgery of a new type and robotic surgery, which ensures the widest possible field of view even in an abdominal cavity and allows universal movement of a viewpoint and, moreover, is capable of grasping the intra-abdominal state in real time and in four dimensions by applying virtual reality technology.

## Claims

1. A photographic device including a camera array in which a plurality of photographic units are arrayed on a leading end of an arm formed to be capable of passing through a tubular body which is inserted into a body from outside the body,
wherein the camera array is displaceably disposed between an alignment location whereat the plurality of photographic units are arrayed along an extension of the arm and a deployment location whereat the plurality of photographic units are arrayed laterally to the arm.

2. The photographic device according to claim 1, wherein the photographic device is provided with a plurality of the camera arrays.

3. The photographic device according to any one of claims 1 and 2, wherein the plurality of photographic units are arrayed in an arcuate manner at the deployment location.

4. The photographic device according to any one of claims 1 to 3, comprising a stereoscopic image generation means for forming a stereoscopic image based on image signals from the two adjacent photographic units.

5. The photographic device according to any one of claims 1 to 4, comprising a three-dimensional model generation means for generating a three-dimensional model of a target object contained in a subject, based on a plurality of subject images at different viewpoints captured by the plurality of photographic units.

6. An image display system comprising:
a photographic device according to any one of claims 1 to 3; and
an image display device which displays a plurality of images at different viewpoints formed based on image signals from a plurality of photographic units provided in the photographic device.

7. The image display system according to claim 6, comprising:
a stereoscopic image generation means for forming a stereoscopic image based on image signals from the two adjacent photographic units; and
a display control means for displaying the stereoscopic image.

8. The image display system according to any one of claims 6 and 7, comprising:
a three-dimensional model generation means for generating a three-dimensional model of a target object contained in a subject, based on the plurality of images at the different viewpoints; and
a display control means for displaying an image of the target object as observed from any user-selected viewpoint, based on the three-dimensional model.
